# FASCICULE DE BREVET EUROPEEN

(11) **EP 1 016 728 B1**
(45) Date de publication et mention de la délivrance du brevet: **20.04.2005**
(21) Numéro de dépôt: 99403287.8
(22) Date de dépôt: 27.12.1999
(51) Int. Cl.: C12P 19/22, C12P 19/14, C12P 19/20, C12P 7/18

(54) **Procédé de fabrication d'un sirop riche en maltose**
Verfahren zur Herstellung eines Sirups mit hohem Gehalt an Maltose
Process for producing a syrup rich in maltose

(30) Priorité: 29.12.1998 FR 9816539
(43) Date de publication de la demande: 05.07.2000
(73) Titulaire: Roquette Frères, 62136 Lestrem (FR)
(72) Inventeur: Caboche, Jean-Jacques, 62131 Drouvin Le Marais (FR)
(74) Mandataire: Touati, Catherine

(56) Documents cités:
- EP-A- 0 816 373
- EP-A- 0 905 256
- FR-A- 2 012 831
- US-A- 4 846 139
- DATABASE WPI Section Ch, Week 9019 Derwent Publications Ltd., London, GB; Class D16, AN 90-144908 XP002114479 & JP 02 092296 A (TOWA KASEI KOGYO KK), 3 avril 1990 (1990-04-03)
- DOYLE E M ET AL: "Mechanisms of action of the maltogenic alpha amylase of Byssochlamys fulva." ENZYME AND MICROBIAL TECHNOLOGY, vol. 22, no. 7, 15 mai 1998 (1998-05-15), pages 612-616, XP002114478 Dep. of Ind. Microbiol., Univ. Coll. Dublin, Belfield, Dublin, Republic of Ireland

## Description

L'invention concerne un procédé de fabrication d'un sirop riche en maltose. Elle concerne aussi l'utilisation d'un sirop riche en maltose obtenu par le procédé conforme à la présente invention pour la fabrication d'un sirop riche en maltitol. Elle concerne encore l'utilisation d'un sirop riche en maltose obtenu par le procédé conforme à la présente invention pour la fabrication de maltitol cristallisé.

On connaît déjà des procédés permettant d'obtenir des sirops riches en maltose. Parmi ces procédés, on peut notamment citer celui décrit par HODGE et Coll. dans "Cereal Chemistry" n° 25, pages 19-30, janvier 1948, et qui comprend une étape de précipitation des dextrines limites par des solutions alcooliques et celui décrit par WOLFROM et THOMPSON dans "Methods in carbohydrate chemistry", 1962, pages 334-335.

D'autres procédés de fabrication de sirops riches en maltose ont encore été proposés comprenant une étape d'adsorption sur charbon des dextrines (US-A-4.194.623), une étape de chromatographie sur zéolithes ou résines cationiques ou anioniques (FR-A-2.510.581), une étape d'ultrafiltration des sirops de maltose (US-A-4.429.122), l'utilisation combinée de plusieurs enzymes différentes, à savoir une α-amylase, une β-amylase et une isoamylase ou une pullulanase (FR-A-2.012.831).

Cette dernière technique présente, par rapport aux précédentes, de nombreux avantages. Elle souffre néanmoins de certains inconvénients, dont notamment celui résidant dans le fait que les saccharifications doivent être effectuées à des teneurs en matières sèches très basses, de l'ordre de 20 g/l, pour obtenir une efficacité d'hydrolyse maximum des enzymes.

Le document FR-A-2.000.580 décrit un procédé de préparation d'un sirop à teneur élevée en maltitol par hydrogénation d'un sirop à teneur élevée en maltose qui est obtenu par liquéfaction d'un lait d'amidon à faible teneur en matières sèches jusqu'à un dextrose-équivalent inférieur à 2, le produit ainsi obtenu étant saccharifié sous l'action d'enzymes spécifiques.

Ce procédé est coûteux, d'un rendement médiocre et donne lieu à des problèmes de contamination bactérienne et à des phénomènes de rétrogradation de l'amylose. En outre, le sirop obtenu contient des proportions de polymères de degré de polymérisation (DP, dans la suite de la description) supérieur ou égal à 4, qui sont gênantes.

Le document JP02092296 décrit un procédé de fabrication de maltose comprenant une étape de saccharification du lait d'amidon en présence d'une beta amylase, suivie d'une étape de mise en contact de la fraction contenent le maltose avec une alpha- amylase maltogénique.

Plus récemment, le document US-A-5.141.859 a proposé un procédé de fabrication d'un sirop à teneur élevée en maltose, mettant en oeuvre deux étapes successives de saccharification. Ce document préconise en effet un procédé comprenant une première étape de saccharification en présence d'une β-amylase et une étape subséquente de saccharification en présence d'une α-amylase maltogénique. Selon ce document, l'α-amylase maltogénique est utilisée, après la première étape de saccharification à la β-amylase, pour hydrolyser les oligosaccharides (de DP3 à DP7) et essentiellement le maltotriose (trisaccharide), en maltose et glucose.

Si l'utilisation d'α-amylase maltogénique permet effectivement et avantageusement d'abaisser la proportion en maltotriose par hydrolyse de ce dernier en maltose et glucose, elle présente néanmoins l'inconvénient majeur de générer des quantités importantes de glucose, et éventuellement de sorbitol en cas d'hydrogénation des hydrolysats. En effet, au glucose résiduel obtenu après saccharification du lait d'amidon liquéfié vient s'ajouter une proportion importante de glucose issu de l'hydrolyse du maltotriose par l'α-amylase maltogénique.

Ces quantités importantes de glucose, donc de sorbitol après hydrogénation, rendent la cristallisation du maltitol plus difficile et conduisent à une baisse de la richesse des cristaux, rendant ceux-ci mal adaptés à certaines applications comme par exemple la fabrication de chocolat.

En outre, la persistance de glucose ou de sorbitol libres dans les sirops de maltose ou de maltitol occasionnent d'autres inconvénients tels qu'un abaissement de la viscosité et de l'humidité relative d'équilibre des produits dans lesquels ils sont incorporés en tant que succédanés du sucre.

Consciente qu'il existe un intérêt croissant pour les produits à très haute teneur en maltose, la Société Demanderesse a conduit de nombreuses recherches dans le but de mettre au point un procédé économique et extrêmement fiable permettant d'obtenir de tels produits.

D'une manière extrêmement simple et particulièrement efficace au regard de tout ce qui a été proposé à ce jour, la Société Demanderesse a constaté que des sirops à très haute teneur en maltose pouvaient être fabriqués en effectuant un tamisage moléculaire d'un lait d'amidon liquéfié et saccharifié de manière à recueillir une fraction enrichie en maltose et une fraction enrichie en glucose puis en mettant en contact ladite fraction enrichie en maltose avec une α-amylase maltogénique.

L'invention propose donc un procédé de fabrication d'un sirop riche en maltose, comprenant les étapes successives consistant à :
(a) effectuer une liquéfaction d'un lait d'amidon ;
(b) effectuer une saccharification du lait d'amidon liquéfié en présence d'une β-amylase et d'au moins une enzyme débranchante choisie dans le groupe constitué par les pullulanases et les isoamylases ;
(c) effectuer un tamisage moléculaire du lait d'amidon liquéfié et saccharifié de manière à recueillir une fraction enrichie en maltose et une fraction enrichie en glucose ;
(d) mettre en contact ladite fraction enrichie en maltose avec une α-amylase maltogénique en vue de l'obtention d'un sirop riche en maltose.

L'invention propose également un procédé de fabrication d'un sirop riche en maltose, comprenant les étapes successives consistant à :
(a) effectuer une liquéfaction d'un lait d'amidon ;
(b) effectuer une saccharification du lait d'amidon liquéfié en présence d'une β-amylase ;
(c) effectuer un tamisage moléculaire du lait d'amidon liquéfié et saccharifié de manière à recueillir une fraction enrichie en maltose et une fraction enrichie en glucose ;
(d) mettre en contact ladite fraction enrichie en maltose avec une α-amylase maltogénique et au moins une enzyme débranchante choisie dans le groupe constitué par les pullulanases et les isoamylases en vue de l'obtention d'un sirop riche en maltose.

Le procédé de fabrication d'un sirop de maltose objet de la présente invention, repose en fait sur un constat simple, négligé jusqu'alors, selon lequel l'action d'une enzyme spécifique sur un substrat déterminé n'est efficace que si les caractéristiques du substrat, en tant que telles, permettent effectivement son action.

Dans le cas présent, une action efficace de l'α-amylase maltogénique ne peut être obtenue qu'à partir d'un lait d'amidon liquéfié et saccharifié ayant une composition particulière, présentant en particulier un spectre glucidique bimodal, à savoir présentant à côté d'une haute teneur en maltose et sensiblement élevée en oligosaccharides, une teneur élevée en glucose.

On cherche donc, dans la présente invention, à ce que les impuretés se présentent sous la forme de glucose plutôt que sous forme d'oligosaccharides, et en particulier de maltotriose, de masse moléculaire proche du maltose.

Le spectre glucidique bimodal particulier du lait d'amidon liquéfié et saccharifié est obtenu, conformément au procédé de l'invention, en effectuant sur ce dernier une étape de tamisage moléculaire.

La première étape du procédé conforme à l'invention est en soi connue. Elle consiste à liquéfier un lait d'amidon dont l'origine botanique peut être quelconque : il peut provenir du blé, du maïs ou de la pomme de terre par exemple.

Ce lait d'amidon ou de fécule est additionné d'acide dans le cas d'une liquéfaction dite acide, ou d'une α-amylase dans le cas d'une liquéfaction enzymatique.

Dans le procédé conforme à l'invention, on préfère effectuer une hydrolyse ménagée du lait d'amidon de façon à obtenir un lait d'amidon liquéfié à faible taux de transformation. Ainsi, les conditions de température, de pH, de taux d'enzyme et de calcium, connues de l'homme du métier, sont déterminées de manière telle qu'elles permettent d'obtenir un DE (Dextrose Equivalent) inférieur à 10, de préférence inférieur à 6, et plus particulièrement inférieur à 4.De préférence, l'étape de liquéfaction est conduite en deux sous-étapes, la première consistant à chauffer, pendant quelques minutes et à une température comprise entre 105 et 108°C, le lait d'amidon en présence d'une α-amylase (type TERMAMYL^{R} 120L commercialisée par la société NOVO) et d'un activateur à base de calcium, la seconde consistant à chauffer le lait d'amidon ainsi traité à une température comprise entre 95 et 100°C pendant une à deux heures.

Une fois l'étape de liquéfaction terminée, dans les conditions de teneur en matières sèches, de pH, de taux d'enzyme et de calcium bien connues de l'homme du métier, on procède à l'inhibition de l'α-amylase. Cette inhibition de l'α-amylase peut se faire de préférence par voie thermique, en procédant en sortie de liquéfaction à un choc thermique de quelques secondes à une température supérieure ou égale à 130°C.

On effectue ensuite la saccharification du lait d'amidon liquéfié au moyen d'une β-amylase telle que celle commercialisée par la société GENENCOR sous la dénomination SPEZYME® BBA 1500.

Lors de cette étape, il convient d'associer à la β-amylase une enzyme hydrolysant spécifiquement les liaisons α-1,6 de l'amidon. Cet ajout d'une enzyme débranchante permet d'une part d'accélérer les réactions d'hydrolyse sans simultanément accélérer les réactions de réversion et, d'autre part, de réduire la quantité d'oligosaccharides hautement branchés résistant normalement à l'action des enzymes maltogéniques.

Cet ajout d'enzyme débranchante peut se faire au moment de l'ajout de la β-amylase ou au moment de l'ajout de l'α-amylase maltogénique.

Selon l'invention, l'enzyme débranchante est choisie dans le groupe constitué par les pullulanases et les isoamylases. La pullulanase est, par exemple, celle commercialisée par la société ABM sous la dénomination PULLUZYME® 750L. L'isoamylase est, par exemple, celle commercialisée par la société HAYASHIBARA.

Avantageusement, le procédé conforme à l'invention est mis en oeuvre en présence d'isoamylase pour laquelle la Société Demanderesse a constaté qu'elle permettait d'obtenir un sirop de maltose présentant une teneur en maltose plus élevée qu'en utilisant une pullulanase.

Dans un mode de réalisation particulier de l'invention, l'étape de saccharification peut être conduite également totalement ou partiellement en présence d'α-amylase fongique en utilisant, par exemple, la SPEZYME® DBA 1500(commercialisée par la société GENENCOR) en lieu et place de la SPEZYME® BBA 1500(commercialisée par la même société).

En fin de saccharification, il est possible d'ajouter un peu d'α-amylase, ce qui améliore généralement les étapes subséquentes de filtration. Les quantités et les conditions d'action des différentes enzymes mises en oeuvre dans les étapes de liquéfaction et de saccharification du lait d'amidon sont généralement celles qui sont recommandées pour l'hydrolyse de l'amidon et sont bien connues de l'homme du métier.

On effectue la saccharification à la β-amylase associée éventuellement à l'enzyme débranchante jusqu'à ce que l'hydrolysat de maltose contienne au moins 75 % en poids de maltose et, de préférence, environ 80 % en poids de maltose. Elle dure au moins 24 heures.

L'hydrolysat ainsi saccharifié est ensuite filtré sur filtre à précouche ou par microfiltration sur membranes, puis déminéralisé et concentré.

A ce stade du procédé conforme à l'invention, on effectue un tamisage moléculaire du lait d'amidon liquéfié et saccharifié de manière à recueillir une fraction enrichie en maltose et une fraction enrichie en glucose. A la suite de quoi, on met en contact la fraction enrichie en maltose avec une α-amylase maltogénique Cette dernière est avantageusement celle commercialisée par la société NOVO, sous les noms Maltogénase® 4000L et NOVAMYL®.

L'étape de tamisage moléculaire mise en oeuvre dans le procédé conforme à l'invention peut consister, par exemple, en une étape de séparation chromatographique ou en une étape de séparation sur membranes.

L'étape de fractionnement chromatographique est effectuée de manière connue en soi, de façon discontinue ou continue (lit mobile simulé), sur des adsorbants du type résines cationiques, ou sur des zéolithes fortement acides, chargées préférentiellement à l'aide d'ions alcalins ou alcalino-terreux tels que le calcium ou le magnésium mais plus préférentiellement à l'aide d'ions sodium.

En lieu et place de l'étape de séparation chromatographique, il est possible, dans le procédé conforme à l'invention, de mettre en oeuvre une étape de séparation par nanofiltration sur membranes. Des membranes de différents diamètres de pores sont fabriquées à partir de nombreux polymères et copolymères du type polysulfones, polyamides, polyacrylonitrates, polycarbonates, polyfuranes, etc.

Des exemples de l'utilisation de telles membranes sont décrits notamment dans les documents US-A-4.511.654, US-A-4.429.122 et WO-A-95/10627.

Selon un mode de réalisation avantageux du procédé conforme à l'invention, la partie non maltose issue des membranes ou de la chromatographie, comportant la fraction enrichie en glucose, est recyclée en amont de l'étape de saccharification.

Grâce au procédé conforme à l'invention qui tire profit des bénéfices obtenus à la fois des étapes d'hydrolyse mises en oeuvre et de l'étape de tamisage moléculaire, il est possible d'obtenir, avec des rendements supérieurs à 90 %, un hydrolysat d'amidon dont la teneur en maltose est supérieure à 95 %, et même supérieure à 98 % lorsqu'une isoamylase est mise en oeuvre dans les étapes d'hydrolyse.

A ce stade du procédé conforme à l'invention, il est éventuellement possible d'effectuer sur l'hydrolysat (ou sirop de maltose) une cristallisation du maltose ou une hydrogénation catalytique.

L'hydrogénation d'un tel hydrolysat s'effectue conformément aux règles de l'art qui conduisent par exemple à la production de sorbitol à partir du glucose.

On peut utiliser pour cette étape aussi bien des catalyseurs à base de ruthénium que des catalyseurs au nickel de RANEY. On préfère cependant utiliser des catalyseurs au nickel de RANEY qui sont moins onéreux.

Dans la pratique, on utilise de 1 à 10 % en poids de catalyseur par rapport à la matière sèche de l'hydrolysat soumis à l'hydrogénation. L'hydrogénation s'effectue de préférence sur un hydrolysat dont la matière sèche est comprise entre 15 et 50 %, dans la pratique voisine de 30 à 45 %, sous une pression d'hydrogène comprise entre 20 et 200 bars. Elle peut être effectuée de manière continue ou discontinue.

Lorsque l'on opère de manière discontinue, la pression d'hydrogène utilisée est généralement comprise entre 30 et 60 bars et la température à laquelle se déroule l'hydrogénation est comprise entre 100 et 150°C. On veille aussi à maintenir le pH du milieu d'hydrogénation par l'addition de soude ou de carbonate de soude par exemple, mais sans dépasser un pH de 9,0. Cette manière de faire permet d'éviter l'apparition de produits de cracking ou d'isomérisation.

On arrête la réaction lorsque la teneur du milieu réactionnel en sucres réducteurs est devenue inférieure à 1 %, de préférence encore inférieure à 0,5 % et plus particulièrement inférieure à 0,1 %.

Après refroidissement du milieu réactionnel, on élimine le catalyseur par filtration et on déminéralise le sirop de maltitol ainsi obtenu sur des résines cationiques et anioniques. A ce stade, les sirops contiennent au moins 93 % de maltitol.

Le sirop de maltitol obtenu à l'étape d'hydrogénation précédente peut alors subir une étape de cristallisation de manière à obtenir du maltitol cristallisé.

Selon un mode de réalisation préféré conforme à l'invention, on met en oeuvre sur le sirop de maltitol obtenu à l'étape d'hydrogénation précédente, la succession des étapes suivantes consistant à :
- concentrer le sirop de maltitol ;
- cristalliser et séparer les cristaux de maltitol formés ;
- effectuer sur les eaux-mères de cristallisation un tamisage moléculaire et, en particulier, un fractionnement chromatographique de manière à obtenir une fraction enrichie en maltitol et une fraction appauvrie en maltitol ;
- recycler la fraction enrichie en maltitol en amont de l'étape de cristallisation ;
- effectuer éventuellement sur la fraction appauvrie en maltitol, une hydrolyse acide et/ou une hydrolyse enzymatique au moyen par exemple d'une amyloglucosidase immobilisée ou non ;
- effectuer éventuellement une hydrogénation de ladite fraction appauvrie en maltitol et hydrolysée en vue d'obtenir un sirop de sorbitol.

De manière surprenante et inattendue, l'utilisation selon l'invention de sirop riche en maltose pour la fabrication de maltitol cristallisé permet de diminuer de façon très importante (jusqu'à 80% par rapport à un procédé conventionnel) les quantités d'eaux-mères (i.e. la fraction appauvrie en maltitol) générées lors de l'étape de tamisage moléculaire en aval de l'étape de cristallisation.

D'autres caractéristiques et avantages de l'invention apparaîtront clairement à la lecture des exemples qui suivent. Ils ne sont toutefois donnés ici qu'à titre illustratif et non limitatif.

### EXEMPLE 1

Un lait d'amidon, à une matière sèche de 31 %, est liquéfié de manière classique à l'aide de 0,2 % de TERMAMYL® 120L (α-amylase commercialisée par la société NOVO) à un pH de 5,7 à 6,5 jusqu'à un DE légèrement inférieur à 4.

On chauffe ensuite le milieu réactionnel pendant quelques secondes à 140°C de manière à inhiber l'α-amylase, puis on ajuste le pH entre 5 et 5,5 et la température à 55°C.

La saccharification est conduite à une matière sèche de 25 %, ou légèrement inférieure, en présence de pullulanase (PULLUZYME® 750L commercialisée par la société ABM) et de β-amylase (SPEZYME® BBA commercialisée par la société GENENCOR) à des doses respectives de 0,1 % et 0,05 % sur matière sèche.

La saccharification, qui dure environ 48 heures, donne un hydrolysat montrant la composition suivante, DP1 : 1,4 %, DP2 : 82,4 %, DP3 : 13,2 %, DP4 et plus : 2,6 %.

L'hydrolysat subit ensuite une purification classique par filtration, décoloration et déminéralisation puis est concentré à environ 20 % de matière sèche et ajusté à pH 5,5.

On procède à une étape de chromatographie continue de l'hydrolysat de maltose ainsi obtenu de la manière suivante.

Quatre colonnes d'un litre de résine PCR 732 sodique thermostatées à 75°C sont assemblées en série et alimentées en continu avec l'hydrolysat de maltose amené à une matière sèche de 60 % en poids, à un débit de 110 ml/h.

En sortie de colonne on récupère les fractions enrichies en maltose de composition suivante : DP1 : 1,5 %, DP2 : 94 %, DP3 : 4,5 %.

Le rendement chromatographique en maltose est de 91,5 %.

Ces fractions sont concentrées à environ 20% de matière sèche et ajusté à pH 5, 5.puis sont mises en contact avec une α-amylase maltogénique (Maltogénase® 4000L commercialisée par la société NOVO) à une dose de 0,3% sur matière sèche. La composition du sirop de maltose obtenu est la suivante : DP1 : 4 %, DP2 : 95,5 %, DP3 : 0,5 %.

### EXEMPLE 2

Le sirop de maltose obtenu dans l'exemple 1 ci-dessus est soumis à une étape de cristallisation du maltose de la manière suivante. On prépare une solution de maltose d'une matière sèche de 75 % en poids à une température de 75°C. On ensemence la solution de maltose avec 5 % en poids de germes de cristaux de maltose et refroidit la solution de 75°C à 40°C, à raison de 0,5°C par heure, en agitant la solution à 50 tours/min dans un cristallisoir à double paroi.

En fin de cristallisation, les cristaux sont séparés de la liqueur mère à l'aide d'une essoreuse centrifuge conventionnelle.

Le rendement de cristallisation est de 50 % en poids exprimé en poids de maltose cristallisé sur le poids de maltose de départ. La pureté en maltose des cristaux récupérés est de 97,5 % sur sec. La teneur en eau est de 5%.

### EXEMPLE 3

Le sirop de maltose issu de l'exemple 1 est déminéralisé puis hydrogéné dans les conditions suivantes :

| | |
|---|---|
| Matière sèche | 40 % |
| Température | 115°C |
| Dose de catalyseur | 5 % P/P sec |
| Pression d'H₂ | 50 bars |

On arrête la réaction quand les sucres réducteurs sont inférieurs à 0,3 %. Le milieu est alors filtré, déminéralisé et concentré à 85 % de matière sèche ; sa composition est :

| | |
|---|---|
| Sorbitol | 5,5 % |
| Maltitol | 94,0 % |
| Supérieurs hydrogénés | 0,5 % |

On procède alors à l'étape de cristallisation par refroidissement de 75 à 25°C, à raison de 0,5°C/heure sous agitation lente, avec amorçage de 6 % P/P sec de maltitol cristallisé de granulométrie comprise entre 200 et 250µm.

Après turbinage, les cristaux sont séchés et présentent une richesse de 99,7 % ; les eaux-mères sont ajustées à 60 % de matière sèche et chromatographiées.

Quatre colonnes d'un litre de résine PCR732, sous forme calcium, thermostatées à 85°C sont assemblées en série et alimentées de façon continue à un débit de 120 ml/h. Le rendement en maltitol est de 90,7 % et la fraction noble (fraction riche en maltitol) présente la composition suivante : sorbitol 4,5 % ; maltitol 95 % ; supérieurs hydrogénés : 0,5 %

La fraction appauvrie en maltitol, contenant 53,5 % de sorbitol, 42,5 % de maltitol et 4 % de supérieurs hydrogénés, est ensuite soumise à une étape d'hydrolyse acide.

L'hydrolyse de la fraction appauvrie est réalisée en continu, sur une résine échangeuse de cations, de type C145 de Purolite, sous forme H⁺ placée dans une colonne thermostatée à 115°C ; en alimentant la colonne à 1 bv/h avec la solution concentrée à 40 %, la composition suivante est obtenue : sorbitol : 70,5 % ; maltitol : 12,3 % ; supérieurs : 0,4 % ; glucose : 16,8 %.

Cette solution est ensuite déminéralisée et hydrogénée dans les conditions suivantes :

| | |
|---|---|
| Matière sèche | 40 % |
| Température | 135°C |
| Dose de catalyseur | 5 % P/Psec |
| Pression d'hydrogène | 50 bars. |

jusqu'à obtenir un taux de sucres réducteurs libres inférieur à 0,1 %.

## Revendications

1. Procédé comprenant les, étapes successives consistant à :
(a) effectuer une liquéfaction d'un lait d'amidon ;
(b) effectuer une saccharification du lait d'amidon liquéfié en présence d'une β-amylase et d'au moins une enzyme débranchante choisie dans le groupe constitué par les pullulanases et les isoamylases ;
(c) effectuer un tamisage moléculaire du lait d'amidon liquéfié et saccharifié de manière à recueillir une fraction enrichie en maltose et une fraction enrichie en glucose ;
(d) mettre en contact ladite fraction enrichie en maltose avec une α-amylase maltogénique en vue de l'obtention d'un sirop riche en maltose.

2. Procédé comprenant les étapes successives consistant à :
(a) effectuer une liquéfaction d'un lait d'amidon ;
(b) effectuer une saccharification du lait d'amidon liquéfié en présence d'une β-amylase ;
(c) effectuer un tamisage moléculaire du lait d'amidon liquéfié et saccharifié de manière à recueillir une fraction enrichie en maltose et une fraction enrichie en glucose ;
(d) mettre en contact ladite fraction enrichie en maltose avec une α-amylase maltogénique et au moins une enzyme débranchante choisie dans le groupe constitué par les pullulanases et les isoamylases en vue de l'obtention d'un sirop riche en maltose.

3. Procédé selon la revendication 1 ou la revendication 2, **caractérisé par le fait qu'**il comprend en outre une étape (e) consistant à cristalliser le sirop riche en maltose de l'étape (d) en vue d'obtenir du maltose cristallisé.

4. Procédé selon la revendication 1 ou la revendication 2 qui comprend en outre une étape (e) consistant à hydrogéner le sirop riche en maltose obtenu à l'étape (d) en vue d'obtenir un sirop riche en maltitol.

5. Procédé selon la revendication 4, qui comprend une étape (f) consistant à cristalliser le sirop riche en maltitol obtenu à l'étape (e) en vue d'obtenir du maltitol cristallisé.

6. Procédé selon la revendication 4, **caractérisée par le fait qu'**il comprend les étapes consistant à :
(f) cristalliser le sirop riche en maltitol obtenu à l'étape (e) ;
(g) effectuer un tamisage moléculaire des eaux-mères de cristallisation de manière à recueillir une fraction enrichie en maltitol et une fraction appauvrie en maltitol ;
(h) recycler ladite fraction riche en maltitol en amont de l'étape de cristallisation ;
(i) effectuer une hydrolyse acide et/ou enzymatique de ladite fraction appauvrie en maltitol ;
(j) effectuer une hydrogénation de ladite fraction appauvrie en maltitol et hydrolysée.

## Patentansprüche

1. Verfahren, umfassend die aufeinanderfolgenden Schritte, die darin bestehen, dass:
(a) eine Verflüssigung einer Stärkemilch vorgenommen wird,
(b) eine Verzuckerung der verflüssigten Stärkemilch in Gegenwart einer β-Amylase und mindestens eines trennenden Enzyms vorgenommen wird, das aus der Gruppe ausgewählt ist, die aus Pullulanasen und Isoamylasen besteht,
(c) eine Molekularsiebung der verflüssigten und verzuckerten Stärkemilch so vorgenommen wird, dass eine hinsichtlich Maltose angereicherte Fraktion und eine hinsichtlich Glucose angereicherte Fraktion gewonnen wird,
(d) die hinsichtlich Maltose angereicherte Fraktion mit einer maltogenen α-Amylase in Kontakt gebracht wird, um einen Sirup mit hohem Maltosegehalt zu erhalten.

2. Verfahren, umfassend die aufeinanderfolgenden Schritte, die darin bestehen, dass:
(a) eine Verflüssigung einer Stärkemilch vorgenommen wird,
(b) eine Verzuckerung der verflüssigten Stärkemilch in Gegenwart einer β-Amylase vorgenommen wird,
(c) eine Molekularsiebung der verflüssigten und verzuckerten Stärkemilch so vorgenommen wird, dass eine hinsichtlich Maltose angereicherte Fraktion und eine hinsichtlich Glucose angereicherte Fraktion gewonnen wird,
(d) die hinsichtlich Maltose angereicherte Fraktion mit einer maltogenen α-Amylase und mindestens einem trennenden Enzym in Kontakt gebracht wird, das aus der Gruppe ausgewählt ist, die aus Pullulanasen und Isoamylasen besteht, um einen Sirup mit hohem Maltosegehalt zu erhalten.

3. Verfahren nach Anspruch 1 oder Anspruch 2, **dadurch gekennzeichnet, dass** es außerdem einen Schritt (e) umfasst, der darin besteht, dass der Sirup mit hohem Maltosegehalt des Schritts (d) kristallisiert wird, um kristallisierte Maltose zu erhalten.

4. Verfahren nach Anspruch 1 oder Anspruch 2, das außerdem einen Schritt (e) umfasst, der darin besteht, dass der im Schritt (d) erhaltene Sirup mit hohem Maltosegehalt hydriert wird, um einen Sirup mit hohem Maltitolgehalt zu erhalten.

5. Verfahren nach Anspruch 4, das einen Schritt (f) umfasst, der darin besteht, dass der im Schritt (e) erhaltene Sirup mit hohem Maltitolgehalt kristallisiert wird, um kristallisiertes Maltitol zu erhalten.

6. Verfahren nach Anspruch 4, **dadurch gekennzeichnet, dass** es die Schritte umfasst, die darin bestehen, dass:
(f) der im Schritt (e) erhaltene Sirup mit hohem Maltitolgehalt kristallisiert wird,
(g) eine Molekularsiebung der Kristallisationsflüssigkeiten so durchgeführt wird, dass eine hinsichtlich Maltitol angereicherte Fraktion und eine hinsichtlich Maltitol verarmte Fraktion gewonnen wird,
(h) die hinsichtlich Maltitol angereicherte Fraktion stromauf des Kristallisationsschritts rezykliert wird,
(i) eine saure und/oder enzymatische Hydrolyse der hinsichtlich Maltitol verarmten Fraktion durchgeführt wird,
(j) eine Hydrierung der hinsichtlich Maltitol verarmten und hydrolysierten Fraktion vorgenommen wird.

## Claims

1. Process comprising the successive steps consisting in:
(a) carrying out liquefaction of a starch milk;
(b) carrying out saccharification of the liquefied starch milk in the presence of a β-amylase and at least one debranching enzyme selected from the group comprising pullulanases and isoamylases;
(c) carrying out molecular sieving of the liquefied and saccharified starch milk so as to collect a fraction enriched in maltose and a fraction enriched in glucose;
(d) bringing said fraction enriched with maltose into contact with a maltogenic α-amylase in order to obtain a maltose-rich syrup.

2. Process comprising the successive steps consisting in:
(a) carrying out liquefaction of a starch milk;
(b) carrying out saccharification of the liquefied starch milk in the presence of a β-amylase;
(c) carrying out molecular sieving of the liquefied and saccharified starch milk so as to collect a fraction enriched in maltose and a fraction enriched in glucose;
(d) bringing said fraction enriched in maltose into contact with a maltogenic α-amylase and at least one debranching enzyme selected from the group comprising pullulanases and isoamylases in order to obtain a maltose-rich syrup.

3. Process according to claim 1 or to claim 2, **characterized by** the fact that it further comprises a step (e) consisting in crystallising the maltose-rich syrup of step (d) in order to obtain crystallised maltose.

4. Process according to claim 1 or claim 2, **characterized by** the fact that it further comprises a step (e) consisting in hydrogenating the maltose-rich syrup obtained at step (d) in order to obtain a maltitol-rich syrup.

5. Process according to claim 4, which comprises a step (f) consisting in crystallising the maltitol-rich syrup obtained at step (e) in order to obtain crystallised maltose.

6. Process according to claim 5, **characterized by** the fact that it comprises the steps consisting in:
(f) crystallising the maltitol-rich syrup;
(g) carrying out molecular sieving of the crystallisation mother liquors so as to obtain a maltitol-rich fraction and a maltitol-poor fraction;
(h) recycling said maltitol-rich fraction upstream of the crystallisation step;
(i) carrying out acid and/or enzymatic hydrolysis of said maltitol-poor fraction;
(j) carrying out hydrogenation of said hydrolysed maltitol-poor fraction.
